# EUROPEAN PATENT APPLICATION

(11) **EP 3 858 980 A1**
(43) Date of publication of application: **04.08.2021**
(21) Application number: 19864328.0
(22) Date of filing: 27.09.2019
(51) Int. Cl.: C12N 5/07

(54) **CULTURE ADDITIVE, CULTURE MEDIUM AND CULTURE METHOD FOR ANIMAL CELLS**

(30) Priority: 28.09.2018 JP 2018184352
(71) Applicant: AJINOMOTO CO., INC., Chuo-ku Tokyo 104-8315 (JP)
(72) Inventor: MATSUMOTO, Takuya, Kawasaki-shi, Kanagawa 210-8681 (JP); OGAWA, Shimpei, Kawasaki-shi, Kanagawa 210-8681 (JP); FUROMITSU, Shumpei, Kawasaki-shi, Kanagawa 210-8681 (JP); OHYA, Yusuke, Kawasaki-shi, Kanagawa 210-8681 (JP); KOSEKI, Kotoe, Kawasaki-shi, Kanagawa 210-8681 (JP); HIGUCHI, Takuya, Kawasaki-shi, Kanagawa 210-8681 (JP); NISHIYAMA, Megumi, Kawasaki-shi, Kanagawa 210-8681 (JP)
(74) Representative: Hoffmann Eitle
(86) International application number: PCT/JP2019/038354
(87) International publication number: WO 2020/067502

(57) **Abstract**

The present invention relates to an additive for culturing animal cells that contains amino acid or glucose, or amino acid and glucose, a medium for culturing animal cells that contains amino acid or glucose, or amino acid and glucose in addition to a medium component, and a method for culturing animal cells that includes culturing the animal cells using the aforementioned medium, or adding the aforementioned additive to a culture.

According to the present invention, the proliferation ability of animal cells can be improved particularly when the animal cells are cultured using a serum-free medium or a low albumin medium.

## Description

### [Technical Field]

The present invention relates to an additive for culturing, a medium for culturing, and a method for culturing animal cells that can improve proliferation ability of animal cells.

### [Background Art]

Many animal cells including stem cells such as embryonic stem cells, and induced pluripotent stem cells have been proliferated and maintained by adhesion culture using human-type recombinant matrix such as Matrigel, vitronectin and laminin as scaffold materials.

However, as a method for efficiently proliferating and culturing a large amount of animal cells for application to research, substance production, medical treatment, and the like, a method for suspension culturing in the state of a cell aggregate is widely used instead of the above-mentioned adhesion culture.

In culturing animal cells, it is generally necessary to add about 5 (w/v)% - 20 (w/v)% of serum to the culture broth to supplement components such as growth factor and the likes.

However, serum is expensive, and contains not less than 500 kinds of proteins including unidentified components. Thus, it is often difficult to study at the molecular level the factors that act on cells and to isolate and purify substances produced from cells. In addition, serum may contain prions, viruses and the like. For culturing animal cells, therefore, a serum-free medium containing no serum or a low albumin medium with a decreased albumin content is often used. When culturing animal cells using a serum-free medium or a low albumin medium, proteins such as transferrin, various growth factors, hormones such as insulin, and the like are added to improve proliferation ability of the cells.

However, the kind and combination of growth factors, hormones, etc. necessary for proliferation vary depending on the type of animal cells to be cultured. It is thus difficult to obtain, at a low cost, an additive capable of promoting the proliferation of various animal cells.

Accordingly, a culture additive for animal cells that can efficiently promote proliferation of various animal cells has been demanded.

### [Summary of Invention]

### [Technical Problem]

Thus, the present invention aims to provide a culture additive, a culture medium and a culture method capable of improving proliferation ability of animal cells particularly when culturing the animal cells using a serum-free medium or a low albumin medium.

### [Solution to Problem]

The present inventors have conducted intensive studies in an attempt to solve the aforementioned problems and found that the proliferation ability of animal cells is improved by adding an amino acid such as tryptophan, serine, cysteine, cystine, methionine, arginine or the like, or glucose, or adding the aforementioned amino acid and glucose, which resulted in the completion of the present invention.

That is, the present invention relates to the following.
[1] An additive for culturing animal cells, comprising an amino acid or glucose, or an amino acid and glucose.
[2] The additive of [1], wherein the amino acid is one or more kinds selected from the group consisting of tryptophan, serine, cysteine, cystine, methionine and arginine.
[3] The additive of [1], wherein the amino acid is one or more kinds selected from the group consisting of tryptophan, serine, cysteine, cystine, methionine, arginine, histidine, isoleucine, leucine, lysine, phenylalanine and valine.
[4] The additive of any of [1] to [3], wherein the animal cell is a stem cell.
[5] The additive of [4], wherein the stem cell is one or more kinds selected from the group consisting of an adult stem cell, an embryonic stem cell, and an induced pluripotent stem cell.
[6] The additive of any of [1] to [5], wherein the additive is for suspension culture of animal cells.
[7] The additive of [6], wherein the suspension culture of animal cells is suspension culture in the state of cell aggregate.
[8] The additive of any of [1] to [5], wherein the additive is for adhesion culture of animal cells.
[9] A medium for culturing an animal cell, comprising an amino acid or glucose, or an amino acid and glucose, in addition to a medium component.
[10] The medium of [9], wherein the amino acid is one or more kinds selected from the group consisting of tryptophan, serine, cysteine, cystine, methionine and arginine.
[11] The medium of [9], wherein the amino acid is one or more kinds selected from the group consisting of tryptophan, serine, cysteine, cystine, methionine, arginine, histidine, isoleucine, leucine, lysine, phenylalanine and valine.
[12] The medium of any of [9] to [11], wherein the medium has a pH of not less than 7.
[13] The medium of any of [9] to [12], wherein the animal cell is a stem cell.
[14] The medium of [13], wherein the stem cell is one or more kinds selected from the group consisting of an adult stem cell, an embryonic stem cell and an induced pluripotent stem cell.
[15] The medium of any of [9] to [14], wherein the medium is for suspension culture of animal cells.
[16] The medium of [15], wherein the suspension culture of animal cells is suspension culture of animal cells in the state of cell aggregate.
[17] The medium of any of [9] to [14], wherein the medium is for adhesion culture of animal cells.
[18] A method for culturing an animal cell, comprising culturing the animal cell in a medium for culturing animal cells supplemented with an amino acid or glucose, or an amino acid and glucose, or adding an amino acid or glucose, or an amino acid and glucose to a culture of the animal cell.
[19] The method of [18], wherein the amino acid is one or more kinds selected from the group consisting of tryptophan, serine, cysteine, cystine, methionine and arginine.
[20] The method of [18], wherein the amino acid is one or more kinds selected from the group consisting of tryptophan, serine, cysteine, cystine, methionine, arginine, histidine, isoleucine, leucine, lysine, phenylalanine and valine.
[21] The method of any of [18] to [20], wherein the animal cell is a stem cell.
[22] The method of [21], wherein the stem cell is one or more kinds selected from the group consisting of an adult stem cell, an embryonic stem cell and an induced pluripotent stem cell.
[23] The method of any of [18] to [22], wherein the method is for suspension culture of animal cells.
[24] The method of [23], wherein the animal cells are suspension-cultured while allowing to form a cell aggregate.
[25] The method of any of [18] to [22], wherein the method is for adhesion culture of animal cells.

### [Advantageous Effects of Invention]

According to the present invention, an additive for culturing animal cells that can preferably improve proliferation ability of animal cells, particularly stem cells, can be provided, and a medium for culturing animal cells, and a method for culturing animal cells that can preferably improve proliferation ability of animal cells can be provided.

### [Brief Description of Drawings]

Fig. 1 shows the influence of the culture additive of the present invention on the proliferation ability of human induced pluripotent stem cell 1210B2 line in suspension culture in Experimental Example 1.
Fig. 2 shows the influence of the medium of the present invention on the proliferation ability of human induced pluripotent stem cell 1210B2 line in adhesion culture in Experimental Example 2.
Fig. 3 shows the influence of the medium of the present invention on the proliferation ability of human induced pluripotent stem cell 201B7 line in adhesion culture in Experimental Example 2.
Fig. 4 shows the influence of the culture method of the present invention on the proliferation ability of human induced pluripotent stem cell 1210B2 line in suspension culture in Experimental Example 3.
Fig. 5 shows the influence of the culture method of the present invention on the proliferation ability of human induced pluripotent stem cell 1231A3 line in suspension culture in Experimental Example 3.

### [Description of Embodiments]

The present invention provides an additive for culturing animal cells (hereinafter to be also referred to as "the additive of the present invention" in the present specification).

As used herein, the "additive for culturing animal cells" refers to an additive suitable for culturing animal cells, and to an additive to be added to a medium or culture when culturing animal cells.

As the animal cell in the present invention, mammal-derived normal cell, stem cell and progenitor cell can be mentioned.

As the mammal-derived normal cell, germ cells such as spermatozoon, ovum and the like, and somatic cell constituting the living body can be mentioned.

Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, adipocyte, mesenchymal cell, epithelial cell, epidermal cell (e.g., keratinocyte, corneocyte etc.), endothelial cell, vascular endothelial cell, hepatocyte, chondrocyte, cumulus cell, nerve cell, glial cell, oligodendrocyte, micro glia, astrocyte, heart cell, esophageal cell, muscle cells (e.g., smooth muscle cell, skeleton muscle cell), pancreatic beta cell, melanocyte and mononuclear cell and the like.

The somatic cell includes, for example, cells collected from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, blood vessel tissue, blood (including cord blood), bone marrow, heart, eye, brain, neural tissue and the like.

The stem cell refers to a cell that has self-renewal ability and the ability to differentiate into another type of cell and can proliferate infinitely.

Examples include adult stem cell such as hematopoietic stem cell, satellite cell, neural stem cell, mesenchymal stem cell, mammary gland stem cell, olfactory mucosa stem cell, neural crest stem cell, hepatic stem cell, pancreatic stem cell, muscle stem cell, germline stem cell, intestinal stem cell, hair follicle stem cell and the like; pluripotent stem cell such as embryonic stem cell (ES cell), embryonic tumor cell, embryonic germ cell, induced pluripotent stem cell (iPS cell) and the like; cancer stem cell and the like.

Progenitor cell is a cell in the process of differentiating from the aforementioned stem cell into a specific somatic cell or germ cell, and satellite cell, pancreatic progenitor cell, vascular progenitor cell, endothelial progenitor cell, hematopoietic progenitor cell (cord blood-derived CD34 positive cell, etc.) and the like can be mentioned.

The additive of the present invention is preferably provided as an additive for culture of stem cells, more preferably an additive for culture of adult stem cells, embryonic stem cells, and induced pluripotent stem cells, further preferably an additive for culture of embryonic stem cells and induced pluripotent stem cells.

The additive of the present invention contains amino acid or glucose, or amino acid and glucose.

The amino acid to be contained in the additive of the present invention is an amphoteric organic compound having an amino group and a carboxyl group, preferably α-amino acid which is a constitutional unit of protein in living organisms. Examples thereof include neutral amino acid having an alkyl group (glycine, alanine, valine, leucine, isoleucine), amino acid having a hydroxy group (serine, threonine), amino acid containing sulfur (methionine, cysteine), amino acid having an amide group (asparagine, glutamine), amino acid having an imino group (proline), amino acid having an aromatic group (phenylalanine, tyrosine, tryptophan), acidic amino acid (aspartic acid, glutamic acid), basic amino acid (arginine, lysine, histidine) and the like.

In addition, cystine, which is a dimer of cysteine, can also be used in the same manner as cysteine because it is reduced to cysteine when added to a medium or culture.

The additive of the present invention may contain one kind of amino acid alone or a combination of two or more kinds of amino acids.

From the aspect of a proliferation ability-improving effect on animal cells, it is more preferable to contain, as the amino acid, one or more kinds selected from the group consisting of tryptophan (2-amino-3-(indolyl)propionic acid), serine (2-amino-3-hydroxypropionic acid), cysteine (2-amino-3-sulfanylpropionic acid), cystine (3,3'-dithio bis (2-aminopropionic acid)), methionine ((S)-4-(methylthio)-2-aminobutyric acid) and arginine (2-amino-5-guanidinopentanoic acid), and it is further preferable to contain 5 kinds of amino acids of tryptophan, serine, cysteine or cystine, methionine, and arginine.

In addition, from the aspect of proliferation ability-improving effect on animal cells, histidine (2-amino-3-(1H-imidazol-4-yl)propionic acid), isoleucine (2-amino-3-methylpentanoic acid), leucine (2-amino-4-methylpentanoic acid), lysine (2,6-diamino hexanoic acid), phenylalanine (2-amino-3-phenylpropanoic acid), and valine (2-amino-3-methylbutanoic acid) are also preferable amino acids in addition to the above-mentioned tryptophan, serine, cysteine, cystine, methionine, and arginine.

Therefore, in another embodiment of the present invention, it is more preferable to contain one or more kinds selected from the group consisting of tryptophan, serine, cysteine, cystine, methionine, arginine, histidine, isoleucine, leucine, lysine, phenylalanine and valine, and further preferable to contain 11 kinds of amino acids of tryptophan, serine, cysteine or cystine, methionine, arginine, histidine, isoleucine, leucine, lysine, phenylalanine and valine.

As the amino acid contained in the additive of the present invention, any of L-form, D-form, and DL-form can be used. Preferred are L-form and DL-form, and further preferred is L-form.

As the above-mentioned amino acid, not only a free form but also a salt form can be used. The terms "amino acid", "tryptophan", "serine", "cysteine", "cystine", "methionine", "arginine", "histidine", "isoleucine", "leucine", "lysine", "phenylalanine" and "valine" in the present specification each represent a concept also encompassing a salt thereof. As the salt form, an acid addition salt, a salt with a base, and the like can be mentioned, and a pharmacologically acceptable salt is preferably selected.

Specific examples of the salt of the above-mentioned amino acid include salts with inorganic base, organic base, inorganic acid, organic acid, salt with amino acid, and the like. Examples of the salt with organic base include salts with alkali metals such as lithium, sodium, potassium and the like, salts with alkaline earth metals such as magnesium, calcium and the like, ammonium salt and the like. Examples of the salt with organic base include salts with alkanol amines such as monoethanolamine, diethanolamine, triethanolamine and the like, salts with heterocyclic amines such as morpholine, piperidine and the like, and the like. Examples of the salt with inorganic acid include salts with hydrohalic acid (hydrochloric acid, hydrobromic acid, hydroiodic acid, etc.), sulfuric acid, nitric acid, phosphoric acid and the like, and the like. Examples of the salt with organic acid include salts with monocarboxylic acids such as formic acid, acetic acid, propanoic acid and the like; salts with saturated dicarboxylic acids such as oxalic acid, malonic acid, malic acid, succinic acid and the like; salts with unsaturated dicarboxylic acids such as maleic acid, fumaric acid and the like; salts with tricarboxylic acids such as citric acid and the like; salts with keto acids such as α-ketoglutar acid and the like, and the like. Examples of the salt with amino acid include salts with aliphatic amino acids such as glycine, alanine and the like; salts with aromatic amino acids such as phenylalanine and the like; salts with basic amino acids such as arginine, lysine and the like; salts with acidic amino acids such as aspartic acid, glutamic acid and the like; salts with amino acids that form lactam such as pyroglutamic acid and the like, and the like.

As the above-mentioned amino acid, a free form is preferable for the purpose of the present invention. In addition, hydrochloride is also used preferably.

In the present invention, the above-mentioned amino acids in a free form and in the form of a salt may be either those extracted and purified from naturally occurring animals and plants, or those obtained by a chemical synthesis method, a fermentation method, an enzymatic method, a gene recombination method, or the like. Commercially available products provided by each company may also be used.

The content of amino acid in the additive of the present invention is generally 0.01 wt% - 100 wt%, preferably 0.1 wt% - 100 wt%, based on the total amount of amino acids.

When any of the amino acids contained in the additive of the present invention is an amino acid in the form of a salt, the content of the above-mentioned amino acid is calculated by converting the amino acid into a free form.

When the additive of the present invention contains five kinds of amino acids, tryptophan, serine, cysteine or cystine, methionine and arginine, these are preferably contained such that the weight ratio of the contents converted to free form (tryptophan:serine:cysteine (when cystine is used, shown by the amount converted to cysteine produced by reduction):methionine:arginine) is 1:0.01 - 100:0.01 - 100:0.01 - 100:0.01 - 100, more preferably 1:0.1 - 10:0.1 - 10:0.1 - 10:0.1 - 10.

When the additive of the present invention contains 11 kinds of amino acids, tryptophan, serine, cysteine or cystine, methionine, arginine, histidine, isoleucine, leucine, lysine, phenylalanine and valine, these are preferably contained such that the weight ratio of the contents converted to free form (tryptophan:serine:cysteine (when cystine is used, shown by the amount converted to cysteine produced by reduction):methionine:arginine:histidine: isoleucine:leucine:lys ine:phenylalanine:valine) is 1:0.01 - 100:0.01 - 100:0.01 - 100:0.01 - 100:0.01 - 100:0.01 - 100:0.01 - 100:0.01 - 100:0.01 - 100:0.01 - 100, more preferably 1:0.1 - 10:0.1 - 10:0.1 - 10:0.1 - 10:0.1 - 10:0.1 - 10:0.1 - 10:0.1 - 10:0.1 - 10:0.1 - 10.

Glucose contained in the additive of the present invention is an aldohexose that exists free in fruits and honey, and is used as a carbon source in culturing animal cells.

In the present invention, glucose to be used may be one isolated and purified from fruits, honey, and the like, or one produced by hydrolysis of starch or the like. Commercially available products provided by each company may also be used.

The content of glucose in the additive of the present invention is generally 1 wt% - 100 wt%, preferably 10 wt% - 100 wt%.

The additive of the present invention may contain either amino acid or glucose, or may contain both.

From the aspect of proliferation ability improving effect on animal cells, it is preferable to contain both amino acid and glucose, more preferably one or more kinds selected from the group consisting of tryptophan, serine, cysteine, cystine, methionine and arginine, and glucose, further preferably tryptophan, serine, cysteine or cystine, methionine and arginine, and glucose.

In another embodiment of the present invention, it is more preferable to contain one or more kinds selected from the group consisting of tryptophan, serine, cysteine, cystine, methionine, arginine, histidine, isoleucine, leucine, lysine, phenylalanine and valine, and glucose, further preferably tryptophan, serine, cysteine or cystine, methionine, arginine, histidine, isoleucine, leucine, lysine, phenylalanine and valine, and glucose.

In the additive of the present invention, amino acid and glucose are preferably contained such that the weight ratio of the total amount of amino acid (converted to free form) and glucose [total amount of amino acid (converted to free form):glucose] is 1:1 - 1:500, more preferably 1:5 - 1:50.

In the present invention, amino acid or glucose or amino acid and glucose may be used as they are as the additive of the present invention, or they may be dissolved or dispersed in a solvent such as water, polyhydric alcohol, or the like and used as a liquid form such as aqueous solution, dispersion or the like, or may be mixed with an additive generally used for formulation such as excipient, binder and the like, and used as an additive in a solid form such as powder, granule, tablet or the like.

In addition, the above-mentioned amino acid or glucose, or amino acid and glucose may be mixed with a part of the medium components described below such as carbohydrate, inorganic salt and the like and prepared as the additive of the present invention.

From the viewpoint that the addition to a medium for culturing animal cells is convenient and blending with a medium is easy, the additive of the present invention is preferably provided in the form of liquid, powder, granule, tablet or the like.

The additive of the present invention is preferably prepared through a sterilization treatment. The method of the sterilization treatment is not particularly limited, and examples thereof include autoclave sterilization at 121°C for 20 min, radiation sterilization, ethylene oxide gas sterilization, filter filtration sterilization, and the like. The method can be appropriately selected according to the form and the like of the additive of the present invention.

The additive of the present invention is added to the components of the below-mentioned medium for culturing animal cells, and used for preparation of a medium for culturing animal cells, or used by adding to the below-mentioned medium for culturing animal cells. Alternatively, the additive of the present invention can also be used by adding to a culture of animal cells.

The additive of the present invention is added to a medium for culturing animal cells or a culture of animal cells such that the concentration of amino acid (concentration after conversion to amino acid in a free form) is generally 0.1 mg/L/day - 900000 mg/L/day, preferably 1 mg/L/day - 10000 mg/L/day, more preferably 1 mg/L/day - 600 mg/L/day, and the concentration of glucose is generally 0.1 g/L/day - 900 g/L/day, preferably 1 g/L/day - 200 g/L/day, more preferably 1 g/L/day - 20 g/L/day.

When the additive of the present invention contains, as amino acid, one or more kinds selected from the group consisting of tryptophan, serine, cysteine, cystine, methionine and arginine, the additive of the present invention is added to a medium for culturing animal cells or a culture of animal cells such that the concentration of tryptophan (concentration after conversion to tryptophan in a free form) is generally 0.1 mg/L/day - 11000 mg/L/day, preferably 1 mg/L/day - 1000 mg/L/day, more preferably 1 mg/L/day - 100 mg/L/day, the concentration of serine (concentration after conversion to serine in a free form) is generally 0.1 mg/L/day - 425000 mg/L/day, preferably 1 mg/L/day - 1000 mg/L/day, more preferably 1 mg/L/day - 100 mg/L/day, the concentration of cysteine or cystine (concentration after conversion to cysteine in a free form) is generally 0.1 mg/L/day - 280000 mg/L/day, preferably 1 mg/L/day - 1000 mg/L/day, more preferably 1 mg/L/day - 100 mg/L/day, the concentration of methionine (concentration after conversion to methionine in a free form) is generally 0.1 mg/L/day - 55000 mg/L/day, preferably 1 mg/L/day - 1000 mg/L/day, more preferably 1 mg/L/day - 100 mg/L/day, and the concentration of arginine (concentration after conversion to arginine in a free form) is generally 0.1 mg/L/day - 150000 mg/L/day, preferably 1 mg/L/day - 2000 mg/L/day, more preferably 1 mg/L/day - 200 mg/L/day.

When the additive of the present invention contains, as amino acid, one or more kinds selected from the group consisting of tryptophan, serine, cysteine, cystine, methionine, arginine, histidine, isoleucine, leucine, lysine, phenylalanine and valine, the additive of the present invention is added to a medium for culturing animal cells or a culture of animal cells such that the concentration of tryptophan (concentration after conversion to tryptophan in a free form) is generally 0.1 mg/L/day - 11000 mg/L/day, preferably 1 mg/L/day - 1000 mg/L/day, more preferably 1 mg/L/day - 100 mg/L/day, the concentration of serine (concentration after conversion to serine in a free form) is generally 0.1 mg/L/day - 425000 mg/L/day, preferably 1 mg/L/day - 1000 mg/L/day, more preferably 1 mg/L/day - 100 mg/L/day, the concentration of cysteine or cystine (concentration after conversion to cysteine in a free form) is generally 0.1 mg/L/day - 280000 mg/L/day, preferably 1 mg/L/day - 1000 mg/L/day, more preferably 1 mg/L/day - 100 mg/L/day, the concentration of methionine (concentration after conversion to methionine in a free form) is generally 0.1 mg/L/day - 55000 mg/L/day, preferably 1 mg/L/day - 1000 mg/L/day, more preferably 1 mg/L/day - 100 mg/L/day, the concentration of arginine (concentration after conversion to arginine in a free form) is generally 0.1 mg/L/day - 150000 mg/L/day, preferably 1 mg/L/day - 2000 mg/L/day, more preferably 1 mg/L/day - 200 mg/L/day, the concentration of histidine (concentration after conversion to histidine in a free form) is generally 0.1 mg/L/day - 41900 mg/L/day, preferably 1 mg/L/day - 1000 mg/L/day, more preferably 1 mg/L/day - 100 mg/L/day, the concentration of isoleucine (concentration after conversion to isoleucine in a free form) is generally 0.1 mg/L/day - 41200 mg/L/day, preferably 1 mg/L/day - 1000 mg/L/day, more preferably 1 mg/L/day - 100 mg/L/day, the concentration of leucine (concentration after conversion to leucine in a free form) is generally 0.1 mg/L/day - 22400 mg/L/day, preferably 1 mg/L/day - 1000 mg/L/day, more preferably 1 mg/L/day - 100 mg/L/day, the concentration of lysine (concentration after conversion to lysine in a free form) is generally 0.1 mg/L/day - 900000 mg/L/day, preferably 1 mg/L/day - 1000 mg/L/day, more preferably 1 mg/L/day - 100 mg/L/day, the concentration of phenylalanine (concentration after conversion to phenylalanine in a free form) is generally 0.1 mg/L/day - 29600 mg/L/day, preferably 1 mg/L/day - 1000 mg/L/day, more preferably 1 mg/L/day - 100 mg/L/day, and the concentration of valine (concentration after conversion to valine in a free form) is generally 0.1 mg/L/day - 85000 mg/L/day, preferably 1 mg/L/day - 1000 mg/L/day, more preferably 1 mg/L/day - 100 mg/L/day.

The medium for culturing animal cells to which the additive of the present invention is added generally contains carbohydrate such as sugar such as glucose, sucrose, maltose or the like, starch or the like as a carbon source, and amino acid, peptide, or protein as a nitrogen source.

Therefore, the medium for culturing animal cells to which the additive of the present invention is added may originally contain amino acids and glucose, and the original concentration of amino acid contained in the medium (concentration after conversion to amino acid in a free form) is generally about 0.1 mg/L - 5 g/L, and the original concentration of glucose contained in the medium is generally about 0.1 g/L - 10 g/L.

The proliferation ability of animal cells can be improved by culturing animal cells in a medium for culturing animal cells prepared by adding the additive of the present invention or by adding the additive of the present invention to the medium for culturing animal cells, or by adding the additive of the present invention to a culture of the animal cells. The additive of the present invention is particularly suitable for improving the proliferation ability of animal cells when culturing the animal cells in a serum-free medium or a low albumin medium.

The additive of the present invention can be used for both suspension culture in which animal cells are proliferated while being suspended in a medium, and adhesion culture in which animal cells are proliferated while being adhered to a culture vessel.

When the suspension culture of animal cells is performed using the additive of the present invention, cell aggregates with a controlled size can be formed, and the suspension culture can be performed in the state of cell aggregate.

The present invention also provides a medium for culturing animal cells (hereinafter to be also referred to as "the medium of the present invention" in the present specification).

The "animal cell" is as described above for the additive of the present invention.

The medium of the present invention is preferably provided as a medium for culturing stem cells, more preferably a medium for culturing adult stem cells, embryonic stem cells, and induced pluripotent stem cells, further preferably a medium for culturing embryonic stem cells and induced pluripotent stem cells.

The medium of the present invention contains amino acid or glucose, or amino acid and glucose, in addition to medium components generally used for culturing animal cells.

The amino acid and glucose to be further contained in the medium of the present invention are as described above for the additive of the present invention.

In the present invention, the amino acid or glucose, or amino acid and glucose may be contained in the form prepared as the above-mentioned additive of the present invention and together with the aforementioned medium component, or may be directly added to the medium component.

In the medium of the present invention, amino acid or glucose, or amino acid and glucose are added to the medium component at a concentration mentioned above for the additive of the present invention.

Examples of the medium component that can be contained in the medium of the present invention include medium components generally used for culturing animal cells. For example, sugar such as glucose, fructose, sucrose, maltose and the like; amino acid such as asparagine, aspartic acid, glutamine, glutamic acid and the like; protein such as albumin, transferrin and the like; peptide such as glycylglycylglycine, soybean peptide and the like; serum; vitamin such as vitamin A, vitamin B group (thiamine, riboflavin, pyridoxine, cyanocobalamin, biotin, folic acid, pantothenic acid, nicotinamide etc.), vitamin C, vitamin E and the like; fatty acid such as oleic acid, arachidonic acid, linoleic acid and the like; lipid such as cholesterol and the like; inorganic salt such as sodium chloride, potassium chloride, calcium chloride, magnesium sulfate, sodium dihydrogen phosphate and the like; trace element such as zinc, copper, selenium and the like; buffering agent such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine) and the like; antibiotic such as amphotericin B, kanamycin, gentamicin, streptomycin, penicillin and the like; cell adhesion factor and extracellular matrix component such as type I collagen, type II collagen, fibronectin, laminin, poly-L-lysine, poly-D-lysine and the like; cytokine and growth factor such as interleukin, fibroblast growth factor (FGF), hepatocyte growth factor (HGF), transforming growth factor (TGF)-α, transforming growth factor (TGF)-β, vascular endothelial growth factor (VEGF), activin A and the like; hormone such as dexamethasone, hydrocortisone, estradiol, progesterone, glucagon, insulin and the like, and the like can be mentioned. An appropriate component can be selected and used according to the type of the animal cells to be cultured.

Since serum may contain unidentified factor, prion, virus and the like, it is preferable that the medium of the present invention be free of a serum as the medium component. In addition, when the medium of the present invention is prepared as a medium for culturing human cells, it is preferable that the medium be free of a component derived from an animal other than human.

In the present invention, moreover, the amino acid or glucose, or amino acid and glucose may be added to a medium widely used for culturing animal cells such as the above-mentioned mammal-derived normal cell, stem cell, progenitor cell and the like, and the resulting medium may be used as the medium of the present invention.

Examples of the medium used for culturing mammalian cell-derived normal cells include Dulbecco's Modified Eagle's Medium (DMEM), Ham's Nutrient Mixture F12, DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB131 medium, William's Medium E, Fischer's Medium, and the like.

Examples of the medium used for culturing stem cells include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free Medium for hES cells (Millipore), PluriSTEM (trade mark) Human ES/iPS Medium (EMD Millipore), NutriStem (registered trade mark) hESC XF medium (Biological Industries Israel Beit-Haemek Ltd.), NutriStem (trade mark) XF/FF Culture Medium (Stemgent), AF NutriStem (registered trade mark) hESC XF medium (Biological Industries Israel Beit-Haemek Ltd.), S-medium (DS Pharma Biomedical), StemFit (registered trade mark) AK03N medium (Ajinomoto Co., Inc.), hESF9 medium, hESF-FX medium, CDM medium, DEF-CS 500 Xeno-Free 3D Spheroid Culture Medium (Cellartis), StemFlex medium (Thermo Fisher Scientific) and the like.

As the medium to be used for culturing progenitor cells, HPGM (trade mark) (Cambrex Corporation), QBSF-60 (Quality Biological, Inc.) and the like can be mentioned.

In the present invention, amino acid or glucose, or amino acid and glucose is/are preferably added to a medium for culturing stem cells, more preferably added to a medium for culturing adult stem cells, embryonic stem cells and induced pluripotent stem cells, further preferably added to a medium for culturing embryonic stem cells and induced pluripotent stem cells.

For the purpose of the present invention, a serum-free medium or low albumin medium is more preferably used. In addition, a medium for culturing human cells preferably does not contain a component derived from an animal other than human (xeno-free medium).

The medium of the present invention may originally contain glucose or amino acid as a medium component, and in this case, the original concentration of amino acid in the medium (concentration after conversion to amino acid in a free form) is generally about 0.1 mg/L - 5 g/L, and the original concentration of glucose is generally about 0.1 g/L - 10 g/L.

Furthermore, from the aspect that it is also used for suspension culture of animal cells, the medium of the present invention is preferably in the form of a liquid such as solution, dispersion or the like.

It is preferable to control the pH of the medium of the present invention to 7 or higher when it is used for culturing animal cells.

The pH of the medium of the present invention can be adjusted by adding a pH adjuster or a buffering agent to the medium components contained in the medium of the present invention or the additive of the present invention to be added to the medium of the present invention.

The pH adjuster or buffering agent is not particularly limited as long as it can control the pH of the medium of the present invention to not less than 7. Examples thereof include sodium hydrogen carbonate, sodium carbonate, phosphoric acid, sodium dihydrogen phosphate, sodium monohydrogen phosphate, potassium dihydrogen phosphate, potassium monohydrogen phosphate, tris hydroxymethylaminomethane and the like. They are appropriately selected and used according to the components contained in the medium.

The pH of the medium of the present invention is measured at the culture temperature by a general method. For example, when it is a liquid medium, the pH can be measured by the glass electrode method, and when it is a solid medium, the pH can be measured using a flat type electrode or a general glass electrode after a pre-treatment such as forming a semi-fluid state and the like.

The medium of the present invention can be prepared by adding a component appropriately selected from the above-mentioned medium components together with amino acid or glucose, or amino acid and glucose to a solvent such as water and the like according to a known composition, and dissolving or dispersing them.

The medium of the present invention can also be prepared by adding amino acid or glucose, or amino acid and glucose to the above-mentioned medium for culturing animal cells which is provided by each company or institution, and dissolving or dispersing them.

Furthermore, the medium of the present invention can also be prepared in a state concentrated relative to the concentration at the time of use, or as a freeze-dried powder, and used by diluting with a solvent such as water and the like, or by dissolving or dispersing in a solvent such as water and the like.

The medium of the present invention is preferably prepared by applying a sterilization treatment as mentioned above.

The medium of the present invention can be used for both suspension culture and adhesion culture of animal cells. When the medium of the present invention is used for suspension culture of animal cells, cell aggregates with a controlled size can be formed, which is preferable for suspension culture in the state of cell aggregates.

Using the medium of the present invention, the proliferation ability of animal cells can be improved both in suspension culture and adhesion culture.

The medium of the present invention is preferably used for culture of stem cells, more preferably for culture of adult stem cells, embryonic stem cells, and induced pluripotent stem cells, and further preferably for culture of embryonic stem cells and induced pluripotent stem cells.

Furthermore, the present invention provides a method for culturing animal cells (hereinafter to be also referred to as "the culture method of the present invention" in the present specification).

The culture method of the present invention includes culturing animal cells in a medium for culturing animal cells supplemented with amino acid or glucose, or amino acid and glucose. Alternatively, the culture method of the present invention includes adding amino acid or glucose, or amino acid and glucose, to a culture of animal cells.

In the present invention, both suspension culture and adhesion culture of animal cells can be performed. Particularly, the culture method of the present invention can form cell aggregates with a controlled size, which is preferable for suspension culture in the state of cell aggregate.

The "medium for culturing animal cells supplemented with amino acid or glucose, or amino acid and glucose" is as described above for the additive of the present invention and the medium of the present invention. In the present invention, amino acid or glucose, or amino acid and glucose to be added to the medium for culturing animal cells may be prepared and added as the above-mentioned additive of the present invention, or may be amino acid or glucose, or amino acid and glucose added directly to the medium for culturing animal cells.

In the culture method of the present invention, amino acid and glucose are each added to the medium for culturing animal cells or culture of animal cells at the concentrations mentioned above for the additive of the present invention.

When adhesion culture of animal cells is performed, a scaffold material is preferably used. As such scaffold material, scaffold materials generally used for adhesion culture of animal cells can be used. For example, a basement membrane matrix such as Matrigel and the like and a human type recombinant matrix such as vitronectin, laminin and the like can be used.

The animal cell is as described above for the additive of the present invention.

In the culture method of the present invention, the culture of animal cells can be performed according to a general method for suspension culture or adhesion culture.

That is, using a culture vessel or culture apparatus such as a cell culture plate, a cell culture flask, a bioreactor or the like as appropriate according to the form of culture (suspension culture or adhesion culture) and the culture scale, animal cells are seeded in the above-mentioned medium of the present invention or a medium for culturing animal cells added with the additive of the present invention and cultured at generally 25°C - 39°C, preferably 33°C - 39°C, in the presence of generally 4% by volume - 10% by volume, preferably 4% by volume - 6% by volume, of carbon dioxide, and in the presence of generally 1% by volume - 25% by volume, preferably 4% by volume - 20% by volume, of oxygen for generally 1 day - 30 days, preferably 2 days - 14 days. The medium is exchanged every 2 - 3 days, or once or plural times per day.

To exchange the medium in the case of suspension culture, the animal cells and the medium may be separated by centrifugation or filtration, and then a new medium may be added to the animal cells. Alternatively, animal cells may be appropriately concentrated by centrifugation or filtration, and then a new medium may be added to the cell concentrate.

The acceleration of gravity (G) during the above-mentioned centrifugation is generally 50G - 1,000G, preferably 100G - 500G, and the size (diameter) of the fine pores in the filter to be used for filtration is generally 10 µm - 200 µm.

In the case of adhesion culture, the medium can be exchanged by removing the medium by suction or the like and adding a new medium to the animal cells.

In the present invention, suspension culture of animal cell can be performed by stirring, shaking, circulation, gas bubbling and the like.

Stirring can be performed using a bioreactor, culture tank with impeller and the like at a stirring rate of generally 10 rpm - 2,000 rpm, preferably 40 rpm - 1,000 rpm.

Shaking can be performed using a shaker or a shaking incubator at a shaking rate of generally 10 rpm - 500 rpm, preferably 50 rpm - 250 rpm.

Circulation can be performed using a peristaltic pump, tubing pump and the like at a flow rate of generally 10 µL/min - 1,000 mL/min, preferably 1 mL/min - 100 mL/min. As the tube for circulation, tube for peristaltic pump, tube for tubing pump and the like made of silicone, Neoprene (chloroprene rubber), Marprene (polypropylene-ethylenepropylene rubber), and the like is used.

Gas bubbling can be performed using various spargers such as micro sparger, filter sparger and the like at a gas flow rate of generally 1 mL/min - 1000 mL/min, preferably 50 mL/min - 200 mL/min.

To efficiently obtain a cell aggregate having a controlled size, it is preferable to perform suspension culture of the animal cells with stirring or shaking.

The animal cells cultured by suspension culture can be recovered by centrifugation or filtration using a filter.

Centrifugation is performed at 50 G - 1,000 G, preferably 100 G - 500 G, for about 1 min - 10 min.

Filtration can be performed using a filter with fine pores with a diameter of about 10 µm - 200 µm.

The animal cells cultured by adhesion culture can be recovered by removing the medium by suction, detaching the cells from the culture vessel by treating with, for example, a trypsin solution or a trypsin and ethylenediaminetetraacetic acid (EDTA) solution, and centrifugation or filtration using a filter.

The cultured and recovered animal cells are preferably preserved using a freezing medium containing a cryoprotective agent such as STEM-CELLBANKER (Nippon Zenyaku Kogyo Co., Ltd.) and the like in liquid nitrogen.

According to the culture method of the present invention, the proliferation ability of animal cells can be improved both in suspension culture and adhesion culture.

The culture method of the present invention is preferably used for culturing stem cells, more preferably used for culturing adult stem cells, embryonic stem cells and induced pluripotent stem cells, and further preferably used for culturing embryonic stem cells and induced pluripotent stem cells.

### [Example]

The present invention is described in more detail in the following by referring to Examples.

### [Example 1] additive for culturing animal cells

D-glucose (Nacalai Tesque Inc.; 16806-25) was used as it is as the additive of the present invention (Example 1).

### [Example 2] additive for culturing animal cells

L-tryptophan (Ajinomoto Co., Inc.), L-serine (Ajinomoto Co., Inc.), L-cysteine (Nippon Protein Co., Ltd.), L-methionine (Ajinomoto Co., Inc.) and L-arginine (Ajinomoto Co., Inc.) were mixed at a weight ratio of 1:1:1:1:2 (L-tryptophan:L-serine:L-cysteine:L-methionine:L-arginine) and used as the additive of the present invention (Example 2).

### [Example 3] additive for culturing animal cells

L-tryptophan (Ajinomoto Co., Inc.), L-serine (Ajinomoto Co., Inc.), L-cysteine (Nippon Protein Co., Ltd.), L-methionine (Ajinomoto Co., Inc.), L-arginine (Ajinomoto Co., Inc.) and D-glucose (Nacalai Tesque Inc.; 16806-25) were mixed at a weight ratio of 1:1:1:1:2:100 (L-tryptophan:L-serine:L-cysteine:L-methionine:L-arginine:D-glucose) and used as the additive of the present invention (Example 3).

### [Experimental Example 1] Evaluation of effect of the additive of the present invention in suspension culture of human induced pluripotent stem cells (iPS)

Using a micro bioreactor (ambr15 (Sartorius); 001-0881), iPS cell 1210B2 line (iPS Academia Japan, Inc.) was seeded at a cell density of 2×10⁵ cells/mL in StemFit (registered trade mark) AK03N medium (Ajinomoto Co., Inc.) (14 mL) containing 10 µM Rho-associated kinase inhibitor (Y-27632) (Fuji Film Wako Pure Chemical Coporation; 034-24024), and cultured with stirring under the conditions of 37°C, pH=7.2, carbon dioxide concentration=5% by volume, dissolved oxygen concentration=20% by volume, stirring speed=300 rpm.

On day 2 of seeding and thereafter, 70% of the medium was exchanged with StemFit (registered trade mark) AK03N medium every day; and the additive of the present invention of Example 1 was added to meet a D-glucose concentration of 2 g/L/day, the additive of the present invention of Example 2 was added to meet an L-tryptophan, L-serine, L-cysteine and L-methionine concentration of 20 mg/L/day each and an L-arginine concentration of 40 mg/L/day, the additive of the present invention of Example 3 was added to meet an L-tryptophan, L-serine, L-cysteine and L-methionine concentration of 20 mg/L/day each, an L-arginine concentration of 40 mg/L/day, and a D-glucose concentration of 2 g/L/day, and the culture was continued.

As a control, the culture was continued by exchanging the medium in the same manner without adding any of the additives.

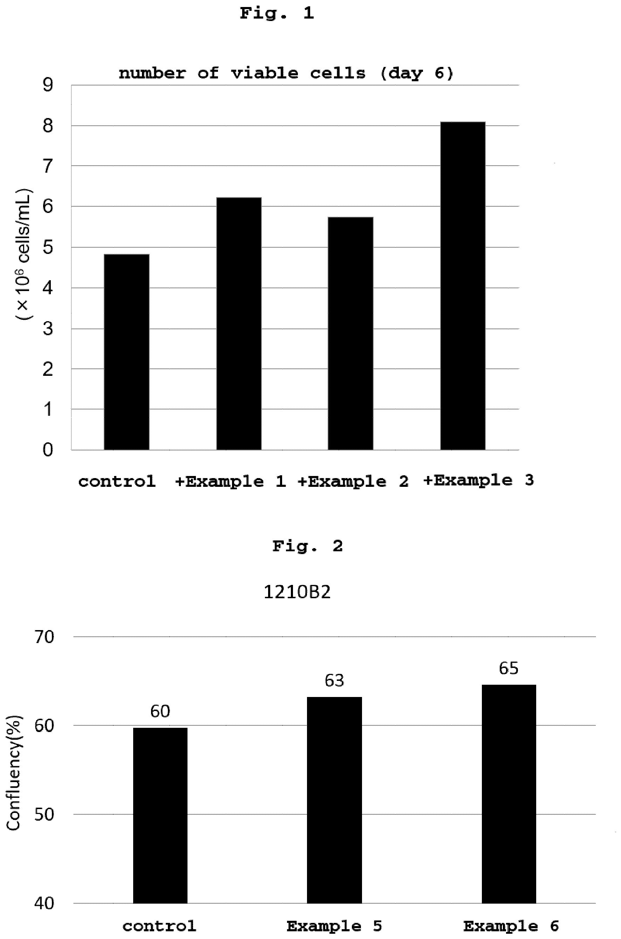

When cultured with the addition of each of the additives of Examples 1 to 3, and in the control, the number of viable cells was measured on day 6 of culture with a cell viability autoanalyzer (Vi-CELL (registered trade mark) XR (Beckman Coulter)). The measurement results are shown in Fig. 1.

As shown in Fig. 1, when the additives of Examples 1 - 3 were respectively added to a culture of iPS cells, the number of viable cells increased as compared to the control.

Therefore, it was confirmed that the addition of 5 kinds of amino acids (L-tryptophan, L-serine, L-cysteine, L-methionine and L-arginine) or glucose improve the proliferation ability of iPS cells.

In addition, it was confirmed that the addition of both the 5 kinds of amino acids and glucose can afford an additive effect of improving the proliferation of iPS cells.

### [Example 4] medium for culturing animal cells

D-glucose (Wako Pure Chemical Industries, Ltd.; 045-31167) was added to StemFit (registered trade mark) AK03N medium (Ajinomoto Co., Inc.) at a concentration of 2 g/L, and the mixture was used as the medium for culturing animal cells of Example 4.

### [Example 5] medium for culturing animal cells

L-tryptophan (Ajinomoto Co., Inc.), L-serine (Ajinomoto Co., Inc.), L-cysteine (Nippon Protein Co., Ltd.) and L-methionine (Ajinomoto Co., Inc.) were added at a concentration of 20 mg/L each, and L-arginine (Ajinomoto Co., Inc.) was added at a concentration of 40 mg/L to StemFit (registered trade mark) AK03N medium (Ajinomoto Co., Inc.), and the mixture was used as the medium for culturing animal cells of Example 5.

### [Example 6] medium for culturing animal cells

L-tryptophan (Ajinomoto Co., Inc.), L-serine (Ajinomoto Co., Inc.), L-cysteine (Nippon Protein Co., Ltd.) and L-methionine (Ajinomoto Co., Inc.) were added at a concentration of 20 mg/L each, L-arginine (Ajinomoto Co., Inc.) was added at a concentration of 40 mg/L, and D-glucose (Wako Pure Chemical Industries, Ltd.; 045-31167) was added at a concentration of 2 g/L to StemFit (registered trade mark) AK03N medium (Ajinomoto Co., Inc.), and the mixture was used as the medium for culturing animal cells of Example 6.

### [Experimental Example 2] Evaluation of effect of the medium of the present invention in adhesion culture of iPS cells

Using 1210B2 line and 201B7 line (iPS Academia Japan, Inc.) as iPS cells, the 1210B2 line was subjected to adhesion culture in each medium of Examples 5 and 6, and the 201B7 line was subjected to adhesion culture in each medium of Examples 4 - 6.

A 12-well plate coated with a basement membrane matrix (iMatrix-511 (Nippi, Inc.; 892012)) was prepared. Each medium (1 mL) of Examples 4 - 6 was added per well, 10,000 cells were seeded in the state of single cell, and cultured under the conditions of 37°C, carbon dioxide concentration=5% by volume, oxygen concentration=20% by volume for 7 days. To each medium was added Rho-associated kinase inhibitor (Y-27632) (Fuji Film Wako Pure Chemical Coporation; 034-24024) at a final concentration=10 µM. The total amount of the medium was changed on days 1, 2, 5 and 6 of culture.

As a control, adhesion culture of iPS cell 1210B2 line and 201B7 line was performed in the same manner using StemFit (registered trade mark) AK03N medium (Ajinomoto Co., Inc.).

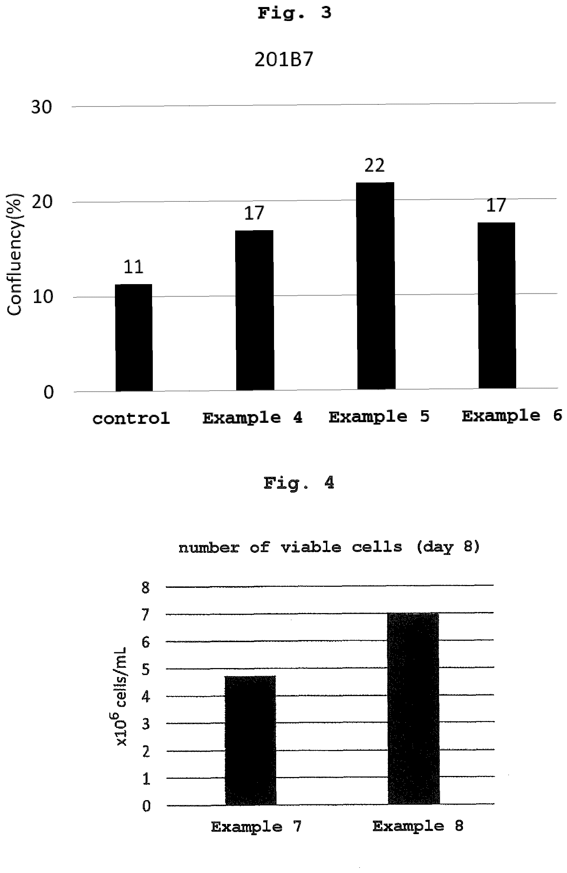

On day 7 of culture, the cell confluency was measured by IncuCyte (Essen BioScience K.K.). Adhesion culture and confluency measurement using each medium were performed 3 times each, and the measurement results are shown in Figs. 2 and 3 in average values.

As shown in Fig. 2, in the adhesion culture of the 1210B2 line, improvement in cell proliferation ability was observed when each medium of Examples 5 and 6 of the present invention was used as compared with the control.

As shown in Fig. 3, in the adhesion culture of the 201B7 line, improvement in cell proliferation ability was observed when each medium of Examples 4 - 6 of the present invention was used.

From the above-mentioned results of Experimental Example 2, also in the adhesion culture of iPS cells, it was suggested that the cell proliferation ability is improved by the addition of five kinds of amino acids (L-tryptophan, L-serine, L-cysteine, L-methionine and L-arginine) or glucose, or the aforementioned five kinds of amino acids and glucose.

### [Examples 7, 8] suspension culture of iPS cells

Using 30 mL single-use bioreactor for iPS cells (ABLE Corporation: BWV-S03A), iPS cell 1210B2 line and 1231A3 line (iPS Academia Japan, Inc.) were seeded at a cell density of 6×10⁵ cells/mL in StemFit (registered trade mark) AK03N medium (Ajinomoto Co., Inc.) supplemented with 10 µM Rho-associated kinase inhibitor (Y-27632) (Fuji Film Wako Pure Chemical Coporation; 034-24024), and cultured by stirring in a CO₂ incubator (37°C, oxygen concentration=20% by volume, carbon dioxide concentration=5% by volume). On day 2 after seeding, 70% of the medium was exchanged with StemFit (registered trade mark) AK03N medium (Ajinomoto Co., Inc.). On day 3 after seeding, the cell suspension (10 mL) was resuspended in 10 mL of fresh StemFit (registered trade mark) AK03N medium (Ajinomoto Co., Inc.) (cell density=0.806×10⁶ cells/mL for 1210B2 line, cell density=0.481×10⁶ cells/mL for 1231A3 line), transferred into a micro bioreactor (ambr15 (Sartorius):001-0881), and stirring culture was continued under the conditions of 37°C, pH=7.2, dissolved oxygen concentration=20% by volume, carbon dioxide concentration=5% by volume, stirring rate=300 rpm. 70% of the medium was exchanged once per day.

In this case, L-tryptophan (Ajinomoto Co., Inc.) (40 mg/L/day), L-serine (Ajinomoto Co., Inc.) (40 mg/L/day), L-cysteine hydrochloride (Nippon Protein Co., Ltd.) (40 mg/L/day), L-methionine (Ajinomoto Co., Inc.) (40 mg/L/day), L-arginine (Ajinomoto Co., Inc.) (160 mg/L/day), D-glucose (Nacalai Tesque Inc.; 16806-25) (4 g/L/day) were added to one group (Example 7), and the aforementioned five kinds of amino acids and glucose, as well as L-histidine (Ajinomoto Co., Inc.) (18.6 mg/L/day), L-isoleucine (Ajinomoto Co., Inc.) (43.7 mg/L/day), L-leucine (Ajinomoto Co., Inc.) (43.7 mg/L/day), L-lysine hydrochloride (Ajinomoto Co., Inc.) (73.1 mg/L/day), L-phenylalanine (Ajinomoto Co., Inc.) (28.4 mg/L/day) and L-valine (Ajinomoto Co., Inc.) (42.3 mg/L/day) were added to the other group and the cells were cultured (Example 8).

### [Experimental Example 3] Evaluation of effect of the culture method of the present invention in suspension culture of iPS cells

The number of viable cells was measured with a cell viability autoanalyzer (Vi-CELL (registered trade mark) XR (Beckman Coulter)) on day 8 of culture of 1210B2 line and on day 7 of culture of 1231A3 line.

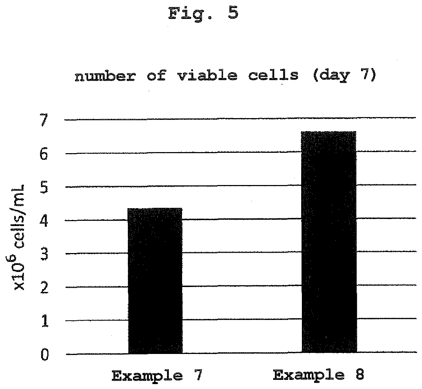

The measurement results of viable cell number of 1210B2 line and 1231A3 line are shown in Figs. 4 and 5, respectively.

As shown in Figs. 4 and 5, the number of viable cells increased as compared with that when resuspended and the cell proliferation was promoted in both the 1210B2 line and 1231A3 line, both by culturing with addition of five kinds of amino acids (L-tryptophan, L-serine, L-cysteine hydrochloride, L-methionine and L-arginine) and glucose (Example 7), and by culturing with addition of 11 kinds of amino acids (L-tryptophan, L-serine, L-cysteine hydrochloride, L-methionine, L-arginine, L-histidine, L-isoleucine, L-leucine, L-lysine hydrochloride, L-phenylalanine and L-valine) and glucose (Example 8). A larger cell proliferation promoting effect was observed when 11 kinds of amino acids and glucose were added (Example 8).

### [Industrial Applicability]

As described in detail above, the present invention can provide an additive for culturing animal cells that can preferably improve the proliferation ability of animal cells, particularly stem cells, and can provide a medium for culturing animal cells and a method for culturing animal cells that can preferably improve the proliferation ability of animal cells.

This application is based on a patent application No. 2018-184352 filed in Japan, the contents of which are incorporated in full herein.

## Claims

1. An additive for culturing animal cells, comprising an amino acid or glucose, or an amino acid and glucose.

2. The additive according to claim 1, wherein the amino acid is one or more kinds selected from the group consisting of tryptophan, serine, cysteine, cystine, methionine and arginine.

3. The additive according to claim 1, wherein the amino acid is one or more kinds selected from the group consisting of tryptophan, serine, cysteine, cystine, methionine, arginine, histidine, isoleucine, leucine, lysine, phenylalanine and valine.

4. The additive according to any one of claims 1 to 3, wherein the animal cell is a stem cell.

5. The additive according to claim 4, wherein the stem cell is one or more kinds selected from the group consisting of an adult stem cell, an embryonic stem cell, and an induced pluripotent stem cell.

6. The additive according to any one of claims 1 to 5, wherein the additive is for suspension culture of animal cells.

7. The additive according to claim 6, wherein the suspension culture of animal cells is suspension culture in the state of cell aggregate.

8. The additive according to any one of claims 1 to 5, wherein the additive is for adhesion culture of animal cells.

9. A medium for culturing an animal cell, comprising an amino acid or glucose, or an amino acid and glucose, in addition to a medium component.

10. The medium according to claim 9, wherein the amino acid is one or more kinds selected from the group consisting of tryptophan, serine, cysteine, cystine, methionine and arginine.

11. The medium according to claim 9, wherein the amino acid is one or more kinds selected from the group consisting of tryptophan, serine, cysteine, cystine, methionine, arginine, histidine, isoleucine, leucine, lysine, phenylalanine and valine.

12. The medium according to any one of claims 9 to 11, wherein the medium has a pH of not less than 7.

13. The medium according to any one of claims 9 to 12, wherein the animal cell is a stem cell.

14. The medium according to claim 13, wherein the stem cell is one or more kinds selected from the group consisting of an adult stem cell, an embryonic stem cell and an induced pluripotent stem cell.

15. The medium according to any one of claims 9 to 14, wherein the medium is for suspension culture of animal cells.

16. The medium according to claim 15, wherein the suspension culture of animal cells is suspension culture of animal cells in the state of cell aggregate.

17. The medium according to any one of claims 9 to 14, wherein the medium is for adhesion culture of animal cells.

18. A method for culturing an animal cell, comprising culturing the animal cell in a medium for culturing animal cells supplemented with an amino acid or glucose, or an amino acid and glucose, or adding an amino acid or glucose, or an amino acid and glucose to a culture of the animal cell.

19. The method according to claim 18, wherein the amino acid is one or more kinds selected from the group consisting of tryptophan, serine, cysteine, cystine, methionine and arginine.

20. The method according to claim 18, wherein the amino acid is one or more kinds selected from the group consisting of tryptophan, serine, cysteine, cystine, methionine, arginine, histidine, isoleucine, leucine, lysine, phenylalanine and valine.

21. The method according to any one of claims 18 to 20, wherein the animal cell is a stem cell.

22. The method according to claim 21, wherein the stem cell is one or more kinds selected from the group consisting of an adult stem cell, an embryonic stem cell and an induced pluripotent stem cell.

23. The method according to any one of claims 18 to 22, wherein the method is for suspension culture of animal cells.

24. The method according to claim 23, wherein the animal cells are suspension-cultured while allowing to form a cell aggregate.

25. The method according to any one of claims 18 to 22, wherein the method is for adhesion culture of animal cells.
